(19) Europäisches Patentamt
European Patent Office
Office européen des brevets

(11) Veröffentlichungsnummer: **0 250 967 B1**

# (12) EUROPÄISCHE PATENTSCHRIFT

(45) Veröffentlichungstag der Patentschrift: **10.07.91**

(51) Int. Cl.[5]: **C07C 69/42**, C07C 69/60, C07C 69/80

(21) Anmeldenummer: **87108443.0**

(22) Anmeldetag: **11.06.87**

## (54) Verfahren zur Herstellung von Hydroxylgruppen aufweisenden Oligoestern und deren Verwendung.

(30) Priorität: **24.06.86 DE 3621039**

(43) Veröffentlichungstag der Anmeldung:
**07.01.88 Patentblatt 88/01**

(45) Bekanntmachung des Hinweises auf die Patenterteilung:
**10.07.91 Patentblatt 91/28**

(84) Benannte Vertragsstaaten:
**AT BE CH DE ES FR GB IT LI NL SE**

(56) Entgegenhaltungen:
**EP-A- 0 124 070**
**DE-A- 2 849 549**
**US-A- 3 576 841**

(73) Patentinhaber: **BAYER AG**

**W-5090 Leverkusen 1 Bayerwerk(DE)**

(72) Erfinder: **Dietrich, Manfred, Dr.**
**Dresdener Strasse 16**
**W-5090 Leverkusen 1(DE)**
Erfinder: **Kapps, Manfred, Dr.**
**Hoppersheider Weg 55**
**W-5060 Bergisch Gladbach 2(DE)**
Erfinder: **König, Klaus, Dr.**
**Zum Hahnenberg 40**
**W-5068 Odenthal(DE)**
Erfinder: **Nast, Roland, Dr.**
**Saarwerdenstrasse 47**
**W-4047 Dormagen 5(DE)**

**EP 0 250 967 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es wird ein Verfahren zur Herstellung von Hydroxylgruppen aufweisenden Oligoestern, welche eine besonders günstige Molekulargewichtsverteilung besitzen, beschrieben, bei dem Umsetzungsprodukte von cyclischen Dicarbonsäureanhydriden mit mehrwertigen Alkoholen oder Dialkanolaminen im Molverhältnis 1:0,5 bis 1,5 anschließend mit Ethylenoxid und/oder Propylenoxid in Äquivalentverhältnissen von Säuregruppen zu Alkylenoxiden von 1:0,8 bis 1,7 bei 80 bis 150 °C, erfindungsgemäß in Gegenwart spezieller Katalysatoren, oxalkyliert werden. Die erfindungsgemäß verwendeten Katalysatoren sind Umsetzungsprodukte von Alkoxiden mit wenigstens 3 C-Atomen mit Ammoniak, Piperidin, Piperazin, $C_2$-$C_6$-aliphatischenPolyaminen, vorzugsweise Diaminen, in denen sämtliche NH-Funktionen alkoxyliert sind.

Die erfindungsgemäßen, relativ niederviskosen Oligoester werden zur Herstellung von Kunststoffen auf Polyisocyanatbasis, insbesondere zur Herstellung von (flammgeschützten)Schaumstoffen, verwendet.

Auf dem Gebiet der Polyurethan-Hartschaumstoffe hat in der letzten Zeit der Flammschutz, insbesondere für den Einsatz auf dem Bausektor, eine zunehmende Bedeutung. In Folge verschärfter gesetzlicher Bestimmungen bezüglich des Brandverhaltens hat sich in den letzten Jahren notwendigerweise auch die Suche nach solchen Ausgangsstoffen verstärkt, aus denen Polyurethan-Hartschaumstoffe erzeugt werden können, welche diese strengen gesetzlichen Normen zu erfüllen vermögen. Zur Erfüllung dieser Normen muß in den seit Jahren üblichen Schaumstoffrezepturen auf Polyetherbasis der Flammschutzmittelanteil sehr stark erhöht werden. Würden hierbei ausschließlich die ökonomisch preiswerten, unfunktionellen Flammschutzmittel wie Tris-β-chlorethylphosphat, Tris-β-chlorisopropylphosphat oder Diphenylkresylphosphat verwendet, so wären die erforderlichen Anteile so groß, daß keine brauchbaren Schaumstoffe mehr entstünden. Es muß daher zusätzlich auf funktionelle, halogenierte und/oder phosphorhaltige Polyole zurückgegriffen werden, die jedoch die Rezepturen stark verteuern. Werden als Polyolkomponente anstelle der seit langem eingesetzten Polyetherpolyole - z.B. auf Zucker, Glycerin oder Sorbit gestartete Polyalkylenoxide im OH-Zahl Bereich 300 bis 550 - jedoch Polyole auf Polyesterbasis im gleichen OH-Zahl Bereich eingesetzt, so sind zur Erfüllung der einschlägigen Brandschutznormen nur wesentlich geringere Mengen an zusätzlichen Flammschutzmitteln notwendig, so daß nun wieder auf die teuren funktionellen Flammschutzmittel ganz verzichtet werden könnte und man mit den preiswerten, unfunktionellen Flammschutzmitteln auskäme.

Polyester, die im genannten OH-Zahl Bereich durch Kondensation von Dicarbonsäuren mit Diolen hergestellt werden, besitzen aufgrund der natürlichen breiten Oligomerenverteilung (Flory-Verteilung) eine für die Verarbeitbarkeit unerwünschte, relativ hohe Viskosität. Außerdem ist der Gehalt an unverestertem, freiem Diol recht hoch. So enthält z.B. ein Polyester aus Phthalsäureanhydrid und Diethylenlykol der OH-Zahl 300 ca. 13 Gew.-% freies Diethylenglykol. Dies führt zu Verarbeitungsschwierigkeiten, sowohl aufgrund der Viskosität als auch auftretender Unverträglichkeiten mit den zum Einsatz kommenden Polyisocyanaten und den als Treibmittel verwendeten Fluorkohlenwasserstoffen.

Die genannten Nachteile bezüglich Viskosität und Verträglichkeit treten verstärkt auf, wenn zur Herstellung der Polyester zusätzlich zu den Diolen auch höherwertige Alkohole eingesetzt werden. Nun sind aber zur Erzielung guter Verschäumungseigenschaften (Aushärtung, Dimensionsstabilität usw.) Funktionalitäten über zwei wünschenswert bzw. notwendig. Es hat aus diesen Gründen in der Vergangenheit nicht an Versuchen gefehlt, derartige Polyester aus Dicarbonsäuren bzw. Dicarbonsäurehalbestern durch Oxalkylierungsreaktionen, d.h. Umsetzung mit Alkylenoxiden herzustellen, weil einerseits hier eine Chance bestand, Produkte mit enger Molgewichtsverteilung zu erhalten, die niedriger viskos als entsprechende Polykondensate sind und keine oder nur wenig freie Alkoholkomponente enthalten. Andererseits sprechen auch ökonomische Aspekte für diesen Weg, da die technisch wohlfeilen, zum Einsatz kommenden Diole wie Ethylenglykol, Propylenglykol oder Diethylenglykol ja aus den Alkylenoxiden durch Wasseranlagerung hergestellt werden und bei der Veresterung dieses Wasser wieder unter Energieverlust abgespalten werden muß.

Die in der Literatur beschriebenen Verfahren erfüllen allerdings die genannten Erwartungen nicht oder nur zum Teil, weil bei den angewandten Bedingungen entweder Veresterungen oder Umesterungen eintreten, so daß letztendlich wieder Produkte mit breiter Molgewichtsverteilung entstehen. Außerdem tritt neben der gewünschten Esterbildung in hohem Maße auch Alkoxylierung der vorhandenen OH-Gruppen ein, so daß als Endprodukte Polyetherester entstehen, die in Polyurethan-Hartschaumstoffen deutlich verminderten Flammschutz ergeben. In den meisten Fällen treten beide Nebenreaktionen zugleich auf. In manchen Fällen ist zum Erreichen eines weitgehenden Umsatzes der Carboxylgruppen ein sehr großer Überschuß an Alkylenoxiden erforderlich, der nach beendeter Umsetzung destillativ entfernt werden muß. Eine solche Verfahrensweise beinhaltet jedoch bei der großtechnischen Herstellung große Nachteile und Gefahren. Aus der sehr umfangreichen Literatur seien die der Erfindung am nächsten liegenden Anmeldungen genannt.

Nach der Lehre der US-A 3 455 886 und US-A 3 459 733 werden ohne Verwendung von Katalysatoren 2 bis 8 Mole Alkylenoxid pro Mol Dicarbonsäurehalbester benötigt, um Säurezahlen unter 1 mg KOH/g zu erreichen. Es wurde daher bald erkannt, daß für eine selektivere Umsetzung eine spezielle Katalyse notwendig ist. In US-A 2 779 783 oder DE-A 1 568 883 wird deshalb die Oxalkylierung in Gegenwart von Alkalimetallhalogeniden, -carbonaten oder -hydroxiden durchgeführt. Alkalimetallhalogenide führen zu Korrosionsproblemen, zudem müssen sie abfiltriert werden. Alkalimetallcarbonate oder -hydroxide verbleiben, sofern sie nicht neutralisiert und filtriert werden, als Carboxylate im Polyester und beeinflussen die Reaktivität entsprechender Polyurethanformulierungen. Eigene Versuche haben außerdem gezeigt, daß bei den notwendigen Reaktionsbedingungen in hohem Maße Umesterungen erfolgen, die zu erhöhten Viskositäten führen, so daß die Endprodukte sich von denen durch Kondensation hergestellten Polyestern kaum unterscheiden (siehe Vergleichsbeispiel 2a). Neben Umesterungen führen Alkalimetallcarbonate oder -hydroxide in hohem Maße zur unerwünschten Etherbildung durch Oxalkylierung der OH-Gruppen, so daß zur Erzielung niedriger Säurezahlen große Alkylenoxidüberschüsse erforderlich sind.

Ferner wird in DE-A 1 248 660 bzw. DE-A 3 201 203 die Umsetzung von Dicarbonsäuren bzw. deren Halbestern mit Alkylenoxiden in Gegenwart von Thiodialkylenglykolen wie Thiodiglykol oder Thiodipropylenglykol beschrieben. Zwar verläuft die Umsetzung weitgehend ohne Nebenreaktionen, doch führt der Einsatz dieser Katalysatoren zu erheblichen Geruchsproblemen sowohl bei Herstellung als auch Weiterverarbeitung dieser Produkte, die von den Schaumstoffherstellern nicht akzeptiert werden. Die Herstellung von Polyestern durch Umsetzung von Carbonsäureanhydriden mit Alkylenoxiden in Abwesenheit von Wasser und in Gegenwart von Glykolen und Katalysatoren wird in der US-A 3 374 208 beschrieben. Als Katalysatoren werden Metallverbindungen genannt, deren Kationen aus einem Zink-, Zinn-, Mangan-, Blei-, Nickel-, Kobalt- oder Cadmium-ion und Anionen aus Sauerstoff-, Chlor-, Acetat-, Butyrat-, Phosphat-, Nitrat-, Stearat-, Oleat-und Naphthenat-ion bestehen.

Bekannt ist außerdem die Veresterung von Carbonsäuren mit Alkylenoxiden in Gegenwart von Katalysatoren, wie z.B. Schwefelsäure, Natriumacetat, Eisen(III)-chlorid u.a. (Methoden der organischen Chemie, Band VIII, Houben-Weyl, Georg Thieme Verlag, Stuttgart, 1952, Seiten 531-533). Chrom(III)-verbindungen (z.B. Chromoctoat) werden, wie in der NL-A 67-01261 und der BE-B 715 201 beschrieben, zur Oxalkylierung von aromatischen Carbonsäuren benutzt. Neben Verfärbungen durch die Chromsalze und andere, die nur unter großen Schwierigkeiten zu entfernen sind, ist auch hier der Ethylenoxidverbrauch zur Erzielung niedriger Säurezahlen zu hoch. In zahlreichen Anmeldungen (FR-A 1 428 204, GB-B 623 669, GB-B 1 060 750, US-A 2 932 622, US-A 2 863 855, US-A 3 414 608, DE-A 3 315 381) ist die Verwendung von tert. Aminen wie Trialkylaminen, Pyridin, Imidazol, N-Methylimidazol, Phosphinen oder Triethanolamin als Katalysator beschrieben, die aber alle einen oder mehrere der genannten Nachteile verursachen. Außerdem können bei einigen Aminen sehr starke Verfärbungen der Produkte auftreten, die wohl ihre Ursache in der Quaternierung des tert. Stickstoffs haben und somit nicht akzeptable Produkte liefern. Aromatische Stickstoffverbindungen wie N-Methylimidazol (siehe Vergleichsbeispiel 2b) sind hervorragende Umesterungskatalysatoren und ergeben daher Produkte mit (unerwünscht) breiter Molgewichtsverteilung.

Es wurde nun überraschend gefunden, daß man ohne die genannten Nachteile Oligoester durch Reaktion von Dicarbonsäurehalbestern mit Alkylenoxiden erhält, wenn man als Katalysatoren Umsetzungsprodukte von Alkoxiden mit wenigstens 3 C-Atomen mit Ammoniak, Piperidin, Piperazin, $C_2$-$C_6$-aliphatischen Polyaminen, vorzugsweise Diaminen, in denen sämtliche NH-Funktionen alkoxyliert sind, einsetzt. Diese Katalysatoren erlauben die Umsetzung der carboxylgruppenhaltigen Ausgangsprodukte mit den Alkylenoxiden unter milden Bedingungen, weil zur vollständigen oder doch weitgehend vollständigen Umsetzung nur geringe Alkylenoxidüberschüsse erforderlich sind. Außerdem werden klare bis schwach gefärbte Produkte erhalten, die keiner Nachbehandlung wie Neutralisation oder Filtration bedürfen. Die Katalysatoren können unbeschadet im Produkt verbleiben, da bei den in Frage kommenden Anwendungen ohnehin oft gleichartige oder ähnliche Produkte in den Formulierungen enthalten sind. Offenbar ist in solchen tert. Aminogruppen enthaltenden Produkten der Stickstoff einerseits sterisch so abgeschirmt, daß es zwar die Umsetzung von Carboxylgruppen mit Alkylenoxiden noch ausreichend katalysiert, aber für Quaternierungsreaktionen und als Katalysator für Veresterungen, Umesterungen oder Oxalkylierungen der OH-Gruppen nicht mehr geeignet ist.

Gegenstand der vorliegenden Erfindung ist somit ein Verfahren zur Herstellung von Hydroxylgruppen enthaltenden Oligoestern im OH-Zahl Bereich 200 bis 600 mg KOH/g durch Umsetzung von Dicarbonsäureanhydriden mit mehrwertigen Alkoholen und/oder Dialkanolaminen im Molverhältnis 1:0,5 bis 1,5, vorzugsweise 1:0,7 bis 1,2 zu den entsprechenden Dicarbonsäurehalbestern und/oder - halbamiden bei Temperaturen von 50 bis 150 °C, vorzugsweise 90 bis 130 °C, und anschließende Oxalkylierung mit Ethylenoxid und/oder Propylenoxid unter Anwendung eines Äquivalentverhältnisses von Säuregruppen zu Alkylenoxiden von 1:0,8 bis 1,7, bei Temperauten von 80-150 °C, vorzugsweise 90 bis 130 °C, dadurch gekennzeichnet, daß die Reaktion Säure/Alkylenoxid unter Verwendung von Umsetzungsprodukte von Alkoxiden mit wenigstens 3 C-Atomen mit Ammoniak, Piperidin, Piperazin, $C_2$-$C_6$-aliphatischenPolyaminen, vorzugsweise Diaminen, in denen sämtliche NH-Funktionen alkoxyliert sind, als Katalysator durchgeführt wird.

Ein weiterer Gegenstand der Erfindung ist ein Verfahren zur Herstellung von Polyurethan-Kunststoffen, vorzugsweise PU-Schaumstoffen einschließlich Polyisocyanurat-Schaumstoffen, welche insbesondere flammwidrige Eigenschaften aufweisen, dadurch gekennzeichnet, daß man die erfindungsgemäßen Oligoester, gegebenenfalls unter Zumischung anderer Polyester- oder Polyetherpolyole und einbaufähiger oder nicht einbaufähiger Flammschutzmittel, bei der üblichen Herstellung von PU-Schaumstoffen nach dem Polyisocyanat-Polyadditionsverfahren verwendet.

Als Ausgangsprodukte für die erfindungsgemäßen Oligoester können eingesetzt werden:
Als cyclische aliphatische und aromatische Dicarbonsäureanhydride Maleinsäureanhydrid, Itaconsäureanhydrid, Citraconsäureanhydrid, Bernsteinsäureanhydrid, Glutarsäureanhydrid, Phthalsäureanhydrid und Tetrahydrophthalsäureanhydrid, vorzugsweise werden Maleinsäureanhydrid und/oder Phthalsäureanhydrid und/oder Glutarsäureanhydrid eingesetzt.

Als mehrwertige Alkohole können zur ringöffnenden Veresterung mit den Anhydriden eingesetzt werden:
Diole wie Ethylenglykol, 1,3- und 1,2-Propandiol, Diethylenglykol, Dipropylenglykol, Tripropylenglykol, 1,3-Pentandiol, 1,6-Hexandiol, 1,4-3,6-Dianhydro-hexite und höherwertige Alkohole wie Glycerin, Trimethylolethan, Trimethylolpropan, Hexan-1,2,6-triol, $\alpha$-Methylglycosid, Pentaerythrit und Sorbit. Vorzugsweise werden jedoch Ethylenglykol, Diethylenglykol, Propylenglykol, Trimethylolpropan, Glycerin und Sorbit eingesetzt. Dabei können die mehrwertigen Alkohole mit Funktionalitäten >2 allein oder im Gemisch mit den Diolen eingesetzt werden.

Als Alkanolamine können allein oder im Gemisch mit mehrwertigen Alkoholen zur ringöffnenden Veresterung mit den Anhydriden eingesetzt werden:
N-Methyl-, -Ethyl- und -Butylethanolamin und Dialkanolamine wie Diethanolamin, Dipropanolamin und Dibutanolamin.

Als Katalysatoren zur Oxalkylierung der Carboxylgruppen werden z.B. Propoxylierungsprodukte von Aminen eingesetzt, die praktisch frei von NH-Gruppen sein müssen, wie handelsübliches Triisopropanolamin oder Propoxylierungsprodukte des Ethylen-, Propylen- oder Hexamethylendiamins mit 4 bis 12, vorzugsweise 5 bis 7 Mol Propylenoxid pro Mol Diamin.

Als Alkylenoxide zur Oxalkylierung der Carboxylgruppen werden Ethylenoxid oder Propylenoxid bzw. Gemisch der beiden Epoxide eingesetzt.

Zur Herstellung der Oligoester wird zunächst aus dem mehrwertigen Alkohol und/oder Dialkanolamin und einem cyclischen Dicarbonsäureanhydrid bei Temperaturen von 50 bis 150 °C, vorzugsweise 90 bis 130 °C, und Reaktionszeiten von 1 bis 10 Stunden, vorzugsweise 2 bis 4 Stunden, der Dicarbonsäurehalbester und/oder das Dicarbonsäurehalbamid hergestellt. Die cyclischen Dicarbonsäureanhydride werden dabei mit den mehrwertigen Alkoholen und/oder Dialkanolaminen im Molverhältnis 1:0,5 bis 1,5, vorzugsweise 1:0,7 bis 1,2, umgesetzt. Die erhaltenen Dicarbonsäurehalbester und/oder -halbamide werden danach in Gegenwart von 0,5 bis 5 Gew.-Teilen eines der erfindungsgemäßen Katalysatoren mit Ethylenoxid und/oder Propylenoxid unter Anwendung eines Äquivalentverhältnisses von Säuregruppen zu Alkylenoxiden von 1:0,8 bis 1,7, vorzugsweise 1:1,0 bis 1,6 bei Temperaturen von 80 bis 150 °C, vorzugsweise 90 bis 130 °C umgesetzt, wobei Säurezahlen von kleiner als 10 angestrebt werden. Die eingesetzte Menge an Alkylenoxid wird dabei so gewählt, daß nach Anwendung der beschriebenen Reaktionsbedingungen kein oder doch nur sehr wenig freies Alkylenoxid mehr zugegen ist. Noch vorhandene Spuren von freiem Alkylenoxid werden unter vermindertem Druck abdestilliert. Manche der eingesetzten cyclischen Dicarbonsäureanhydride neigen bei der zur Ringöffnung nötigen Temperatur zur Sublimation. Deshalb werden die zur Oxalkylierung benötigten, erfindungsgemäß zu verwendenden Katalysatoren bevorzugt schon bei der Ringöffnung eingesetzt, da sie eine schnellere Halbesterbildung bei niedrigen Temperaturan ermöglichen.

Die Verfahrensprodukte stellen je nach Rezeptur mehr oder wenig viskose, helle Flüssigkeiten dar. Das erfindungsgemäße Verfahren erlaubt es an sich unter schonenden Bedingungen und mit wenig Ethylenoxidüberschuß Säurezahlen unter 1 zu erreichen. Für die Anwendung als Polyolkomponente in Polyurethan- und/oder Polyisocyanuratschaumstoffen sind jedoch Säurezahlen bis 10 zulässig. Die erfindungsgemäßen Produkte zeichnen sich durch niedrige Viskositäten aus. Diese kommen dadurch zustande, daß sich die Oligoester nicht wie bei den Polykondensaten im statistischen Gleichgewicht befinden, sondern eine enge Molgewichtsverteilung aufweisen. Überraschenderweise zeigen die erfindungsgemäßen Produkte, trotz der in ihnen enthaltenden Katalysatoren, bei Temperaturen bis 130 °C über längere Zeiträume keine Tendenz unter Viskositätserhöhung zum statistischen Gleichgewicht zu equilibrieren, wie dies bei Verwendung nicht erfindungsgemäßer, bekannter Katalysatoren üblicherweise eintritt. Die Oligoester eignen sich in hervorragender Weise als Ausgangsprodukte für Polyurethan-Kunststoffe, bei denen üblicherweise Polyhydroxylverbindungen mit hohen OH-Zahlen eingesetzt werden, z.B. Beschichtungen, Verklebungen, Duromere, Sandwichwerkstoffe usw. Haupteinsatzgebiet sind jedoch (vorzugsweise flammgeschützte) Polyurethan-Hartschaumstoffe mit unterschiedlichen Polyisocyanuratgehalten.

Zur Herstellung von Polyurethanen, vorzugsweise Polyurethanhartschaumstoffen, werden neben den Oligoestern und den erfindungsgemäßen Katalysatoren eingesetzt:

Polyisocyanate:

Aliphatische, cycloaliphatische, araliphatische, heterocyclische und besonders aromatische Di- und/oder Polyisocyanate, wie sie z.B. von W.Siefken in Justus Liebigs Annalen der Chemie, 562, Seiten 75 bis 136, beschrieben werden, beispielsweise solche der Formel $Q(NCO)_n$, in der n = 2 bis 4, vorzugsweise 2 und Q einen aliphatischen Kohlenwasserstoffrest mit 2 bis 18, vorzugsweise 6 bis 12 C-Atomen, einen cycloaliphatischen Kohlenwasserstoffrest mit 4 bis 20, vorzugsweise 5 bis 11 C-Atomen, einen aromatischen Kohlenwasserstoffrest mit 6 bis 20, vorzugsweise 6 bis 13 C-Atomen, oder einen araliphatischen Kohlenwasserstoffrest mit 8 bis 15, vorzugsweise 8 bis 13 C-Atomen bedeuten, z.B. solche Polyisocyanate wie sie in der DE-A 2 832 253, Seiten 10 bis 11, beschrieben werden. Besonders bevorzugt werden die technisch leicht zugänglichen Polyisocyanate, z.B. das 2,4- und/oder 2,6-Toluylendiisocyanat sowie beliebige Gemische dieser Isomeren ("TDI"), Diphenylmethan-diisocyanate (4,4'-und/oder 2,4'. und/oder 2,2'-Isomere), Polyphenyl-polymethylen-polyisocyanate, wie sie durch Anilin-Formaldehyd-Kondensation und anschließende Phosgenierung hergestellt werden ("rohes MDI") und "modifizierte Polyisocyanate", die z.B. Carbodiimidgruppen, Urethangruppen, Allophanatgruppen, Isocyanuratgruppen, Harnstoffgruppen und/oder Biuretgruppen aufweisen; insbesondere solche modifizierten Polyisocyanate, die sich vom 2,4- und/oder 2,6-Toluylendiisocyanat und bevorzugt vom 4,4'- und/oder 2,4'-Diphenylmethandiisocyanat ableiten. Werden nur zweifunktionelle höhermolekulare Verbindungen und gegebenenfalls nur zweifunktionelle, weitere niedermolekulare Kettenverlängerungsmittel verwendet, so setzt man bevorzugt modifizierte Polyisocyanate mit einer Funktionalität von mehr als 2,0 ein bzw. verwendet Tri-und/oder höherfunktionelle Polyisocyanate.

Ferner werden gegebenenfalls als Ausgangsmaterialien zur Polyurethan-Herstellung sogenannte Kettenverlängerungsmittel oder Vernetzer, d.h. Verbindungen mit mindestens 2 gegenüber Isocyanaten reaktionsfähigen Wasserstoffatomen und einem Molekulargewicht von 18 bis 399 mitverwendet. Auch in diesem Fall versteht man hierunter vorzugsweise Hydroxylgruppen und/oder Aminogruppen und/oder Thiolgruppen und/oder Carboxylgruppen und/oder Hydrazidgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanaten reaktionsfähige Wasserstoffatome auf. Beispiele werden hierfür in der DE-A 2 832 253, Seiten 19-20, beschrieben. Genannt seien Wasser, Hydrazin, Ethylenglykol, Butandiol-1,4, Trimethylolpropan, Formit-Gemische oder Adipinsäuredihydrazid.

In Mengen bis ca. 80 Gew.-%, bezogen auf die erfindungsgemäßen Oligoesterpolyole können als höhermolekulare Co-Polyolkomponente Verbindungen mit mindestens zwei gegenüber Isocyanaten reaktionsfähigen Wasserstoffatomen von einem Molekulargewicht in der Regel von 400 bis 10000 mitverwendet werden. Hierunter versteht man neben Aminogruppen, Thiogruppen oder Carboxylgruppen aufweisenden Verbindungen vorzugsweise Hydroxylgruppen aufweisende Verbindungen, insbesondere 2 bis 8 Hydroxylgruppen aufweisende Verbindungen, speziell solche vom Molekulargewicht 600 bis 6000, vorzugsweise 1500 bis 4000, vorzugsweise aber 2 bis 4 Hydroxylgruppen aufweisende Polyester, Polyether, Polythioether, Polyacetale, Polycarbonate und Polyesteramide, wie sie für die Herstellung von homogenen und zellförmigen Polyurethanen an sich bekannt sind und wie sie z.B. in der DE-A 2 832 253, Seiten 11 bis 18, beschrieben werden. Besonders bevorzugt sind Polyether, die durch Addition von einem oder mehreren Alkylenoxiden (Ethylenoxid und besonders Propylenoxid) an zwei- oder mehrwertige "Starter" (Propylenglykol, Glycerin, Sorbit, Formose, Saccharose, Triethanolamin, Trimethylolpropan) erhalten werden, sowie Polyether, welche Polyadditionsprodukte aus Diisocyanaten und Hydrazin und/oder Diaminen und/oder Glykolen oder Polymerisate und/oder Pfropfpolymerisate, vorzugsweise aus Styrol und Acrylnitril dispergiert oder gelöst enthalten. Die bevorzugten Polyether haben dabei eine mittlere Funktionalität über 2,0.

Gegebenenfalls werden Hilfs- und Zusatzmittel wie leichtflüchtige anorganische, jedoch bevorzugt organische Substanzen als Treibmittel, Katalysatoren der an sich bekannten Art, wie tert. Amine, Zinn(II)- und Zinn(IV)-Verbindungen, oberflächenaktive Zusatzstoffe, wie Emulgatoren und Schaumstabilisatoren, Reaktionsverzögerer, z.B. sauer reagierende Stoffe wie Salzsäure oder organische Säurehalogenide, ferner Zellregler der an sich bekannten Art wie Paraffine, Fettalkohole oder Dimethylpolysiloxane sowie Pigmente oder Farbstoffe der an sich bekannten Art, ferner Stabilisatoren gegen Alterungs-, Licht- unmd Witterungseinflüsse, Weichmacher und fungistatisch und bakteriostatisch wirkende Substanzen, sowie Füllstoffe zugesetzt. Diese gegebenenfalls mitzuverwendenden Hilfs- und Zusatzstoffe werden beispielsweise in der DE-A 2 732 292, Seiten 21 bis 24, ausführlich beschrieben. Weitere Beispiele der Hilfs-und Zusatzmittel werden im Kunststoffhandbund, Band VII, herausgegeben von Vieweg und Höchtlen, Carl-Hanser-Verlag, München 1966, auf den Seiten 103 bis 113, sowie im Kunststoffhandbuch, Band VII, herausgegeben von Becker und Braun, Carl-Hanser-Verlag, München 1983, auf den Seiten 92 bis 111, beschrieben.

Als erfindungsgemäße Katalysatoren werden Umsetzungsprodukte von Alkoxiden mit wenigstens 3 C-Atomen mit Ammoniak, oder niederen aliphatischen oder cycloaliphatischen Diaminen, insbesondere solchen mit 2 bis 6 C-Atomen, eingesetzt, bei denen alle NH-Funktionen vollständig propoxyliert sind, vorzugsweise (vollständig) propoxylierte Produkte des Ammoniaks, Ethylendiamins, 1,2-Propylendiamins oder 1,6-Hexamethylendiamins.

Anstelle von propoxylierten Produkten können auch andere, z.B. vom Butylenoxid oder Styroloxid abgeleitete Produkte eingesetzt werden.

Geeignete Verbindungen sind beispielsweise Triisopropanolamin, Tetraisopropanol-ethylendiamin, -propylendiamin, -butylendiamin, -hexamethylendiamin, -methylpentandiamin, -dimethylbutandiamin, sowie höhere Homologe des Ethylendiamins wie Di-ethylentriamin, Triethylentetramin, Tetraethylenpentamin sowie höhere Homologe des Propylendiamins wie Dipropylendiamin. Weniger bevorzugt können auch 2-Hydroxypropyl-piperidin oder besser Bis-(2-hydroxypropyl)-piperazin oder Bis-(2-hydroxypropyl)-2,5-dimethylpiperazin verwendet werden.

Mit den erfindungsgemäßen Oligoestern hergestellte Hartschaumstoffe finden Verwendung als Dämmplatten, als Sandwich-Elemente mit verschiedenen Deckschichten, als Ortschaumstoffe wie Spritzschaumstoffe oder nach dem Überschichtungsverfahren hergestellten Schaumstoffe, als Sonnenkollektorfüllungen, als Rohrisolierung, als Füll-und Montageschaumstoffe und Blockschaumstoffe.

Ihre Herstellung erfolgt nach üblichen, kontinuierlichen oder diskontinuierlichen Verfahren der Polyurethanverarbeitung wie z.B. der Doppelbandtechnik, Spritz- oder Gießverfahren, mit Hoch- oder Niederdruck-Verschäumungsmaschinen, wobei ihre relativ niedrigen Viskositäten Vorteile, insbesondere bei der maschinellen Verarbeitung bieten.

Das erfindungsgemäße Verfahren wird durch die folgenden Beispiele näher erläutert.

## Beispiele 1 bis 14 und Vergleichsbeispiele

Allgemeine Herstellungsvorschrift

In einem für Umsetzungen mit Alkylenoxiden geeigneten Reaktor werden unter Rühren in einer Stickstoffatmosphäre der mehrwertige Alkohol und/oder das Dialkanolamin bzw. ein Gemisch von mehrwertigen Alkoholen und in der Regel der Katalysator bei Raumtemperatur oder, falls die Alkohole nicht flüssig sind, bei erhöhter Temperatur vorgelegt, das cyclische Dicarbonsäureanhydrid - flüssig oder fest - eingetragen und rasch auf 90 bis 130 °C (je nach Viskosität des entstehenden Halbesters) erhitzt und bei dieser Temperatur in der Regel 2 h umgesetzt. Danach wird, sofern der Katalysator nicht schon in der ersten Stufe zugesetzt wird, nach dessen Zugabe bei 90 bis 130 °C das Alkylenoxid unter einem Stickstoffdruck von 1 bis 3 bar zudosiert. Nach beendeter Zugabe erfolgt eine Nachreaktion bei 100 bis 130 °C von mindestens 5 h, danach werden evtl. Spuren von freiem Alkylenoxid destillativ unter vermindertem Druck entfernt. In der folgenden Tabelle 1) sind als Beispiele 14 Rezepturen, die resultierenden erfindungsgemäßen Oligoester und 3 Vergleichsbeispiele, die unter Verwendung literaturbekannter Katalysatoren hergestellt wurden, gegenübergestellt. Die darin verwendeten Abkürzungen haben folgende Bedeutung:

| | | | |
|---|---|---|---|
| PSA | Phthalsäureanhydrid | EG | Ethylenglykol |
| MSA | Maleinsäureanhydrid | DEG | Diethylenglykol |
| BSA | Bernsteinsäureanhydrid | PG | Propylenglykol |
| | | Gly | Glycerin |
| EO | Ethylenoxid | | |
| PO | Propylenoxid | | |

Katalysator A Umsetzungsprodukt von Ethylendiamin mit 5 Mol PO

Katalysator B Umsetzungsprodukt von Propylendiamin mit 7 Mol PO

Katalysator C Triisopropanolamin

Tabelle 1

| Beispiel | Carbonsäure-anhydrid | mehrwert. Alkohol | Molverhält. COOH/OH | Katalysator*) Gew.-% | | Alkylenoxid Molverh. COOH/Alkylenoxid | OH-Zahl | Säurezahl | $\eta 25^0$ (mPas) |
|---|---|---|---|---|---|---|---|---|---|
| 1 | PSA | DEG | 1:0,6 | 1 | A | EO 1:1,2 | 252 | 1,5 | 20900 |
| 2 | PSA | DEG | 1:0,8 | 1 | A | EO 1:1,3 | 310 | 0,8 | 5900 |
| 3 | PSA | DEG | 1:0,8 | 1 | A | PO 1:1,3 | 287 | 3,7 | 8860 |
| 4 | PSA | DEG | 1:1,5 | 1 | A | EO 1:1,5 | 430 | 0,1 | 780 |
| 5 | PSA | EG | 1:0,7 | 0,5 | C | EO 1:1,0 | 320 | 4,1 | 20100 |
| 6 | PSA | EG | 1:0,8 | 1 | C | EO 1:1,3 | 350 | 3,1 | 9800 |
| 7 | PSA | EG | 1:1,0 | 0,5 | C | EO 1:1,4 | 410 | 1,2 | 5300 |
| 8 | PSA | Gly | 1:1,0 | 1 | B | EO 1:1,7 | 520 | 0,3 | 45800 |
| 9 | PSA | DEG/Sorbit 0,75 0,25 | 1:1,0 | 1 | B | PO 1:1,6 | 460 | 2,5 | 41900 |
| 10 | PSA | DEG/Sorbit 0,6 0,2 | 1:0,8 | 1 | B | EO 1:1,5 | 425 | 3,3 | 54500 |
| 11 | MSA | DEG | 1:0,6 | 0,5 | A | EO 1:1,3 | 298 | 1,5 | 2460 |
| 12 | PSA | Diethanol-amin | 1:1 | 0,5 | C | EO 1:1,2 | 306 | 9,4 | 85300 |
| 13 | BSA | DEG/Di-ethanolamin 0,5 0,5 | 1:1 | 0,5 | A | EO 1:1,2 | 302 | 4,1 | 20340 |
| 14 | BSA | PG | 1:0,8 | 1 | B | PO 1:1,3 | 375 | 3,2 | 4250 |
| Vergl. Bsp. | | | | | | | | | |
| 2a | wie 2 | | 1:0,8 | 1 | KCl | EO 1:1,3 | 308 | 2,6 | 8810 |
| 2b | | | 1:0,8 | 0,2 | Methyl-imidazol | EO 1:1,3 | 307 | 0,2 | 8930 |
| 9a | wie 9 | | 1:1,0 | 1 | LiCl | PO 1:1,6 | 461 | 0,8 | 84130 |

*) bezogen auf Endprodukt

In den angefügten Gelchromatogrammen 1-4 zu dem Beispiel 2 mit entsprechenden Vergleichsbeispielen nach literaturbekannten Verfahren wird die Ausnahmestellung der erfindungsgemäßen Katalysatoren belegt.

Das Gelchromatogramm 1 des Halbesters zu Beispiel 2 (siehe Fig. 1) zeigt neben wenig Diethylenglykol (DEG) im wesentlichen nur die Umsetzungsprodukte von 1 Mol Phthalsäureanhydrid und 1 Mol Diethylenglykol sowie das Umsetzungsprodukt von 2 Mol Phthalsäureanhydrid und 1 Mol Diethylenglykol.

Das Gelchromatogramm 2 des Oligoesters Beispiel 2 (siehe Fig. 2) zeigt im wesentlichen die ursprüngliche enge Molgewichtsverteilung.

Die Gelchromatogramme 3 und 4 der Vergleichsbeispiele 2a und 2b (siehe Fig. 3 und 4) zeigen bei sonst gleicher Rezeptur wie Beispiel 2 eine durch Umesterung, Veresterung und Veretherung stark verbreitete Molgewichtsverteilung, die zu der ermittelten erhöhten Viskosität führt.

Verwendungsbeispiele

In den Tabellen 2 und 3 sind beispielhafte Rezepturen aufgeführt, wobei Tabelle 2 die Herstellung von Polyurethan- bzw. Polyurethan/harnstoff/-Hartschaumstoffen und Tabelle 3 die Verwendung der erfindungsgemäßen Polyole zur Herstellung von Polyisocyanurat-Strukturen enthaltenden Hartschaumstoffen beschreibt.

Es wurden jeweils die in den Tabellen angegebenen Oligoester und Polyethertypen manuell verschäumt, wobei die Rohstoffe zunächst innig miteinander vermischt wurden und nach Zugabe der aufgeführten Isocyanatmenge noch 10 bis 15 sec. verrührt und dann in eine offene Form gegossen wurden.

Es entstanden freie Schaumstoffe der Abmessung 30 cm x 30 cmx 30 cm, deren Aushärtung nach einem manuellen Verfahren bestimmt wurde, weshalb die Ergebnisse nur relativ zueinander bewertet werden können. Das Brandverhalten wurde nach DIN 4102 geprüft.

Ausgangsrohstoffe

MDI

Als Isocyanate wurden handelsübliche Polyphenyl-polymethylen-polyisocyanate, die durch Phosgenierung von Anilin-Formaldehyd-Kondensaten erhalten wurden und NCO-Gehalte von 31 Gew.-% aufweisen, verwendet (Desmodur 44V20 und Desmodur 44V70/Bayer AG)

Beispiele 2, 3 und 9 erfindungsgemäße Oligoester aus vorstehender Tabelle 1

Vergleichspolyether

Handelsübliche Polyetherpolyole auf Basis Sucrose-Propylenglykol-Ethylenglykol-propylenoxid der OH-Zahlen 460 (Desmophen® VP PU 1240 = Polyether-Polyol 1) und 470 (Desmophen® 4034 B = Polyether-Polyol 2) der Bayer AG/Leverkusen

Flammschutzmittel

Diphenyl-kresyl-phosphat: Disflamoll® DPK der Bayer AG

Aktivatoren

- Dimethylcyclohexylamin: Desmorapid® 726 b von RheinChemie, Rheinau
- PIR-Aktivator: Trimerisierungskatalysator auf Basis Kaliumacetat.

Tabelle 2

Rezeptur in Gew.-Teilen

| | | | |
|---|---|---|---|
| Polyether-Polyol 1 | 20 | 20 | 20 |
| Beispiel 2 | 80 | | |
| Beispiel 3 | | 80 | |
| Beispiel 9 | | | 80 |
| Wasser | 2,1 | 2,1 | 2,1 |
| Stabilisator | | | |
| VP AC 3378 | 1,8 | 1,8 | 1,8 |
| Desmorapid® 726 b | 1 | 1 | 1 |
| R 11 | 43 | 43 | 43 |
| Desmodur® 44V20 | 123 | 123 | 152 |
| (Ergebnisse): | | | |
| Startzeit (s) | 26 | 23 | 27 |
| Abbindezeit (s) | 84 | 85 | 85 |
| Aushärtung (min) | 10,5 | 10,5 | 8 |
| Rohdichte ($kg/m^3$) | 20 | 20 | 21 |

(R 11 = Trifluormethan)

Tabelle 3

| Rezeptur (in Gew.-Teilen) | | | | | | |
|---|---|---|---|---|---|---|
| Polyether-Polyol 2 | 60 | 60 | 60 | 60 | 60 | 60 |
| Beispiel 2 | 20 | 40 | | | | |
| Beispiel 3 | | | 20 | 40 | | |
| Beispiel 9 | | | | | 20 | 40 |
| Disflamoll® DPK | 20 | 20 | 20 | 20 | 20 | 20 |
| Stabilisator VP AC 3278 | 1 | 1 | 1 | 1 | 1 | 1 |
| Desmorapid® 726 b | 1 | 1 | 1,2 | 1 | 1,7 | 1,7 |
| PIR-Aktivator | 2,5 | 2,5 | 2,7 | 2,5 | 3,7 | 3,7 |
| R 11 | 40 | 40 | 40 | 40 | 40 | 40 |
| Desmodur® 44V70 | 300 | 300 | 300 | 300 | 300 | 300 |
| Kennzahl | 368 | 314 | 368 | 314 | 338 | 270 |
| **Ergebnisse:** | | | | | | |
| Startzeit (s) | 33 | 30 | 34 | 34 | 35 | 32 |
| Abbindezeit (s) | 89 | 71 | 98 | 102 | 103 | 90 |
| Rohdichte ($kg/m^3$) | 50 | 51 | 50 | 51 | 51 | 50 |
| Baustoffklasse nach DIN 4102 | B2 | B2 | B2 | B2 | B2 | B2 |

## Ansprüche

1. Verfahren zur Herstellung von Hydroxylgruppen aufweisenden Oligoestern mit OH-Zahlen von 200 bis 600 mg KOH/g durch Umsetzung von cyclischen Dicarbonsäureanhydriden mit mehrwrtigen Alkoholen und/oder Dialkanolaminen im Mol.-Verh. 1:0,5 bis 1,5, vorzugsweise 1:0,7 bis 1,2, zu den entsprechenden Dicarbonsäurehalbestern - und/oder -halbamiden bei Temperaturen von 50 bis 150 °C, vorzugsweise 90 bis 130 °C, und anschließende Oxalkylierung der Carboxylgruppen mit Ethylenoxid und/oder Propylenoxid unter Anwendung eines Äquivalentverhältnisses von Säuregruppen zu Alkylenoxiden von 1:0,8 bis 1,7, vorzugsweise 1:1,0 bis 1,6, bei Temperaturen von 80 bis 150 °C, vorzugsweise 90 bis 130 °C, dadurch gekennzeichnet, daß die Oxalkylierung unter Verwendung von Umsetzungsprodukten von Alkoxiden mit wenigstens 3 C-Atomen mit Ammoniak, Piperidin, Piperazin, $C_2$-$C_6$-aliphatischen Polyaminen, vorzugsweise Diaminen, in denen sämtliche NH-Funktionen alkoxyliert sind, als Katalysatoren durchgeführt wird.

2. Verfahren nach Anspruch 1, dadurch gekennzeichnet, daß als Katalysator 0,5 bis 5 Gew.-Teile der Propoxylierungsprodukte des Ammoniaks, Ethylen-, Propylen- oder Hexamethylendiamins pro 100 Gew.-Teile Endprodukt verwendet wird.

3. Verfahren nach Anspruch 1, dadurch gekennzeichnet, daß als cyclisches Dicarbonsäureanhydrid Phthalsäureanhydrid und/oder Maleinsäureanhydrid und/oder Glutarsäureanhydrid verwendet wird.

4. Verfahren nach Anspruch 1, dadurch gekennzeichnet, daß als mehrwertige Alkohole Diole vom Molgewicht 62 bis 182 verwendet werden.

5. Verfahren nach Anspruch 1, dadurch gekennzeichnet, daß als mehrwertige Alkohole drei bis sechswertige Alkohole vom Molgewicht 92 bis 182 gegebenenfalls im Gemisch mit den Diolen gemäß Anspruch 4 verwendet werden.

6. Verwendung der nach den Ansprüchen 1 bis 5 hergestellten Polyester als Ausgangsprodukte zur Herstellung von Kunststoffen auf Polyisocyanatbasis, vorzugsweise PU-Schaumstoffen einschließlich Polyisocyanurat-Schaumstoffen, durch Umsetzung von organischen Polyisocyanaten mit höhermolekularen Verbindungen mit gegenüber NCO-reaktiven Gruppen mit reaktiven H-Atomen, z.B. OH-, $NH_2$-, NHR-, COOH-und/oder SH-Gruppen, vorzugsweise höhermolekularen Polyhydroxylverbindungen, gegebenenfalls Wasser und/oder niedermolekularen Kettenverlängerungsmitteln und/oder Vernetzern in Gegenwart der in der PU-Chemie üblichen Hilfs- und Zusatzstoffe, Katalysatoren einschließlich Trimerisierungskatalysatoren, dadurch gekennzeichnet, daß man als höhermolekulare Polyhydroxylverbindungen die erfindungsgemäßen Oligoester gegebenenfalls unter Zumischung anderer Polyester- oder Polyetherpolyole und einbaufähiger und nicht einbaufähiger Flammschutzmittel einsetzt.

## Claims

1. A process for the production of oligoesters containing hydroxyl groups having an OH value of from 200 to 600 mg KOH/g by reaction of cyclic dicarboxylic anhydrides with polyfunctional alcohols and/or dialkanolamines in a molar ratio of 1:0.5 to 1.5 and preferably in a molar ratio of 1:0.7 to 1.2 to form the corresponding dicarboxylic acid semiesters and/or semiamides at temperatures of from 50 to 150 °C and preferably at temperatures of from 90 to 130 °C and subsequent alkoxylation of the carboxyl groups with ethylene oxide and/or propylene oxide using an equivalent ratio of acid groups to alkylene oxides of 1:0.8 to 1.7 and preferably 1:1.0 to 1.6 at temperatures of from 80 to 150 °C and preferably at temperatures of from 90 to 130 °C, characterized in that the alkoxylation reaction is carried out using reaction products of alkoxides containing at least 3 carbon atoms with ammonia, piperidine, piperazine, $C_{2-6}$ aliphatic polyamines, preferably diamines, in which all the NH functions are alkoxylated, as catalysts.

2. A process as claimed in claim 1, characterized in that propoxylation products of ammonia, ethylene, propylene or hexamethylenediamine are used as catalysts in a quantity of from 0.5 to 5 parts by weight to 100 parts by weight of end product.

3. A process as claimed in claim 1, characterized in that phthalic anhydride and/or maleic anhydride and/or glutaric anhydride is used as the cyclic dicarboxylic anhydride.

4. A process as claimed in claim 1, characterized in that diols having a molecular weight of from 62 to 182 are used as the polyhydric alcohols.

5. A process as claimed in claim 1, characterized in that trihydric to hexahydric alcohols having a molecular weight of from 92 to 182, optionally in admixture with the diols according to claim 4, are used as the polyhydric alcohols.

6. The use of the polyesters produced by the process claimed in claims 1 to 5 as starting products for the production of polyisocyanate-based plastics, preferably PU-foams, including polyisocyanurate foams, by reaction of organic polyisocyanates with compounds of relatively high molecular weight containing NCO-reactive groups with reactive H-atoms, for example OH, $NH_2$, NHR, COOH and/or SH groups, preferably polyhydroxyl compounds of relatively high molecular weight, optionally water and/or low molecular weight chain-extending agents and/or cross-linking agents, in the presence of the auxiliaries and additives and catalysts, including trimerization catalysts, normally used in polyurethane chemistry, characterized in that the oligoesters according to the invention, optionally with addition of other polyester or polyether polyols and incorporable and non-incorporable flameproofing agents, are used as the relatively high molecular weight polyhydroxyl.compounds.

## Revendications

1. Procédé de préparation d'oligoesters contenant des groupes hydroxy, d'indice de OH 200 à 600 mg de KOH/g, par réaction d'anhydrides d'acides dicarboxyliques cycliques avec des alcools polyvalents et/ou des dialcanolamines, à un rapport molaire de 1 : 0,5 à 1,5, de préférence de 1 : 0,7 à 1,2, donnant les hémiesters - et/ou hémi-amides - correspondants d'acides dicarboxyliques, à des températures de 50 à 150 ° C, de préférence de 90 à 130°C, suivie de l'oxyalkylation des groupes carboxyle par l'oxyde d'éthylène et/ou l'oxyde de propylène à un rapport de 1 : 0,8 à 1,7, de préférence de 1 : 1,0 à 1,6 entre les équivalents de groupes acides et les équivalents d'oxydes d'alkylène, à des températures de 80 à 150 ° C, de préférence de 90 à 130 ° C, caractérisé en ce que l'oxyalkylation est effectuée en utilisant en tant que catalyseurs des produits de réaction d'alcoxydes contenant au moins trois atomes de carbone avec l'ammoniac, la pipéridine, la pipérazine, des polyamines aliphatiques, de préférence des diamines en $C_2$-$C_6$, dans lesquels toutes les fonctions NH sont alcoxylées.

2. Procédé selon la revendication 1, caractérisé en ce que l'on utilise en tant que catalyseurs, en quantité de 0,5 à 5 parties en poids pour 100 parties en poids du produit final, les produits de propoxylation de l'ammoniac, de l'éthylène-, de la propylène- ou de l'hexaméthylène-diamine.

3. Procédé selon la revendication 1, caractérisé en ce que l'on utilise en tant qu'anhydrides d'acides dicarboxyliques cycliques, l'anhydride phtalique et/ou l'anhydride maléique et/ou l'anhydride glutarique.

4. Procédé selon la revendication 1, caractérisé en ce que l'on utilise, en tant qu'alcools polyvalents, des diols de poids moléculaire 62 à 182.

5. Procédé selon la revendication 1, caractérisé en ce que l'on utilise, en tant qu'alcools polyvalents, des alcools tri-à hexa-valents de poids moléculaire 92 à 182, éventuellement en mélange avec des diols selon la revendication 4.

6. Utilisation des polyesters préparés selon les revendications 1 à 5, en tant que produits de départ de la préparation de résines synthétiques à base de polyisocyanates, de préférence des mousses de polyuréthannes, y compris des mousses de polyisocyanurates, par réaction de polyisocyanates organiques avec des composés à haut poids moléculaire contenant des groupes à atomes d'hydrogène réactifs, par exemple des groupes OH, $NH_2$, NHR, COOH et/ou SH, réactifs à l'égard des groupes NCO, de préférence des composés polyhydroxylés à haut poids moléculaire, le cas échéant de l'eau et/ou des agents d'allongement des chaînes et/ou agents réticulants à bas poids moléculaire, en présence des produits auxiliaires et additifs usuels de la chimie des polyuréthannes, de catalyseurs, y compris des catalyseurs de trimérisation, caractérisée en ce que l'on utilise en tant que composés polyhydroxylés à haut poids moléculaire les oligoesters selon l'invention, éventuellement en mélange avec d'autres polyester- ou polyétherpolyols et des agents ignifugeants combinables ou non combinables chimiquement.

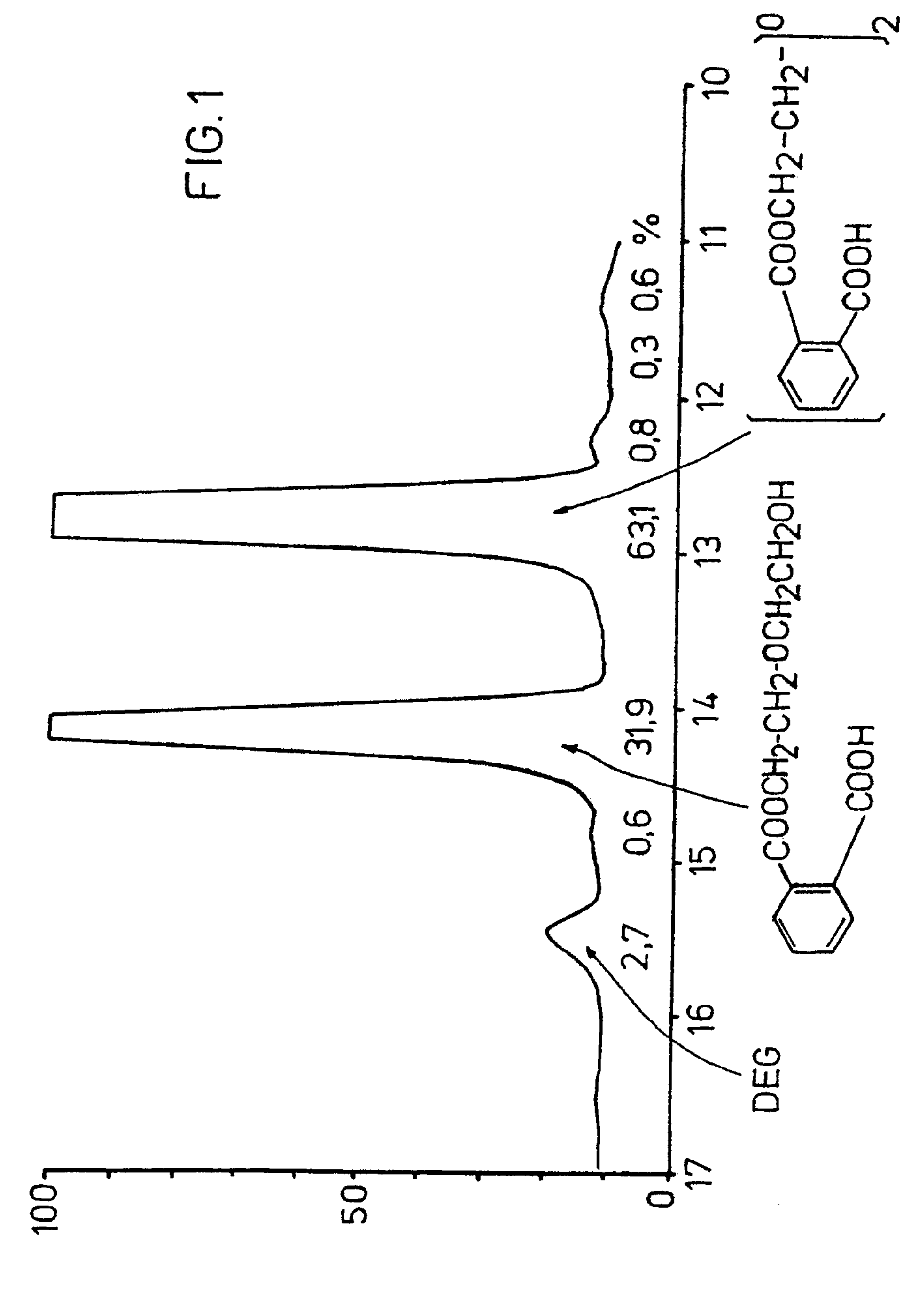


FIG. 1

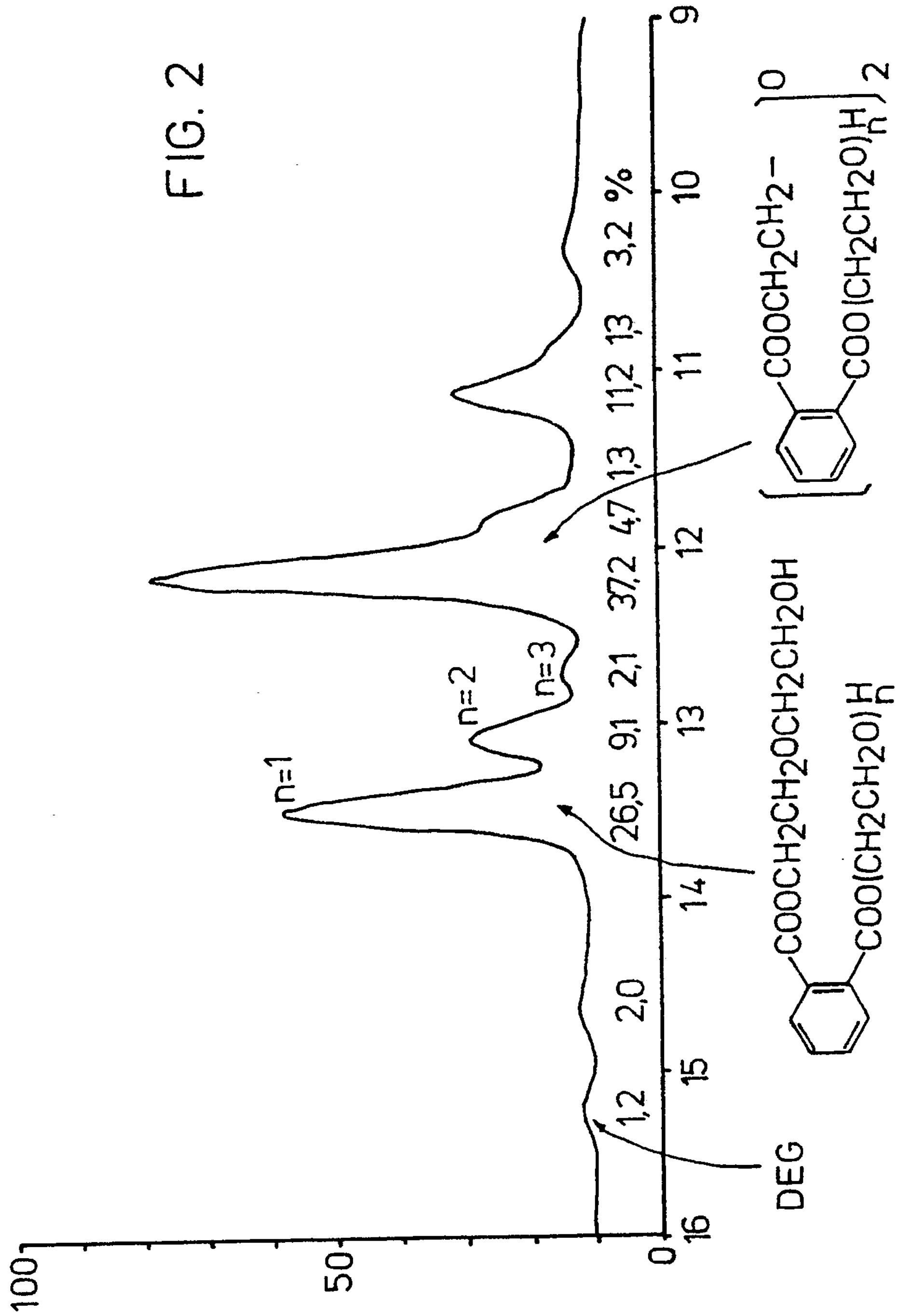
100
50
0
16
15
14
13
12
11
10
9
1,2
2,0
26,5
9,1
2,1
37,2
4,7
1,3
11,2
1,3
3,2 %
n=1
n=2
n=3
DEG

FIG. 2

FIG. 3

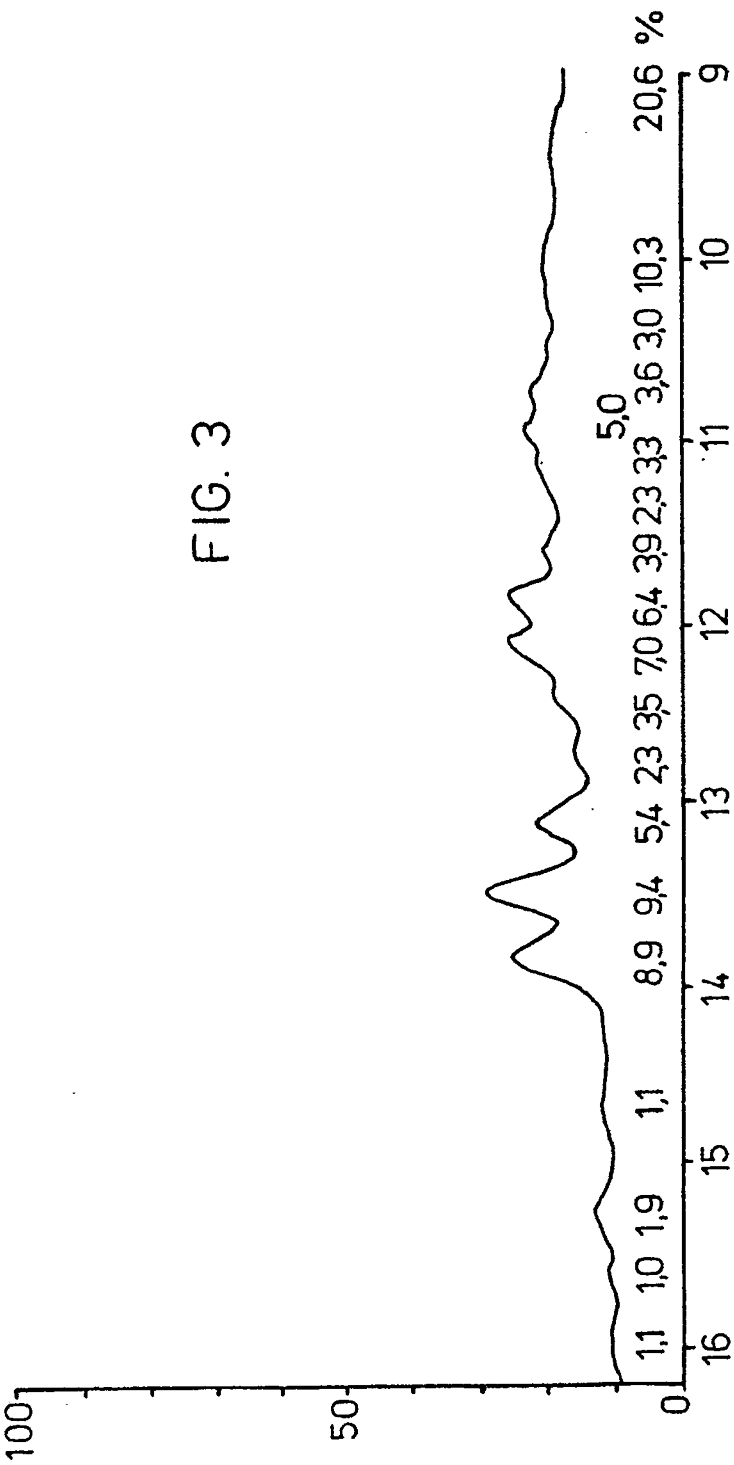
100
50
0
16
15
14
13
12
11
10
9
%
1,1 1,0 1,9 1,1 8,9 9,4 5,4 2,3 3,5 7,0 6,4 3,9 2,3 3,3 5,0 3,6 3,0 10,3 20,6

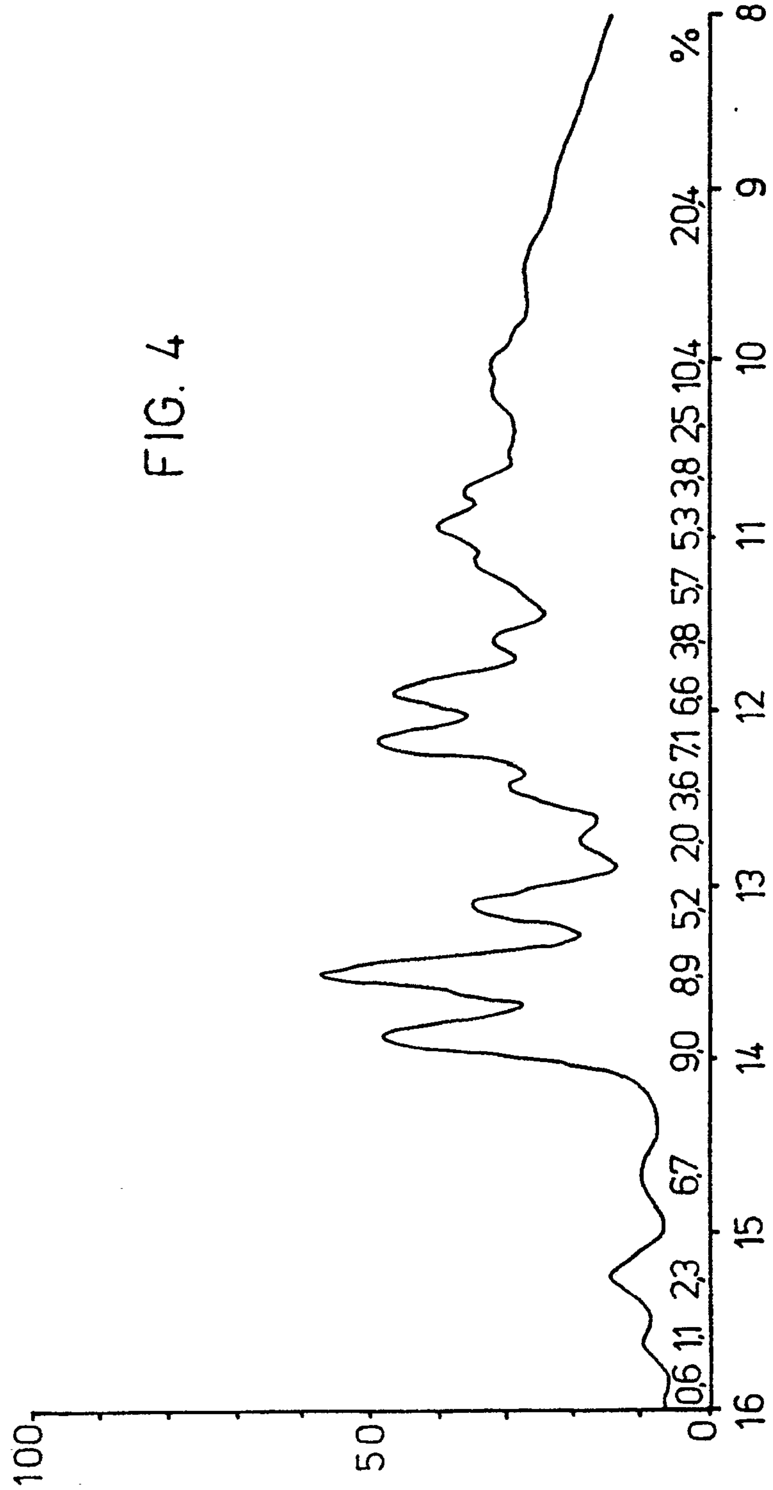
100
50
0
16
15
14
13
12
11
10
9
8
0,6 1,1 2,3 6,7 9,0 8,9 5,2 2,0 3,6 7,1 6,6 3,8 5,7 5,3 3,8 2,5 10,4 20,4 %

FIG. 4